# EUROPEAN PATENT SPECIFICATION

(11) **EP 2 411 534 B1**
(45) Date of publication and mention of the grant of the patent: **01.06.2016**
(21) Application number: 10710320.2
(22) Date of filing: 23.03.2010
(51) Int. Cl.: C12Q 1/68

(54) **METHOD FOR DIAGNOSING OR PREDICTING A NON SYNDROMIC AUTOSOMAL RECESSIVE OPTIC ATROPHY, OR A RISK OF A NON SYNDROMIC AUTOSOMAL RECESSIVE OPTIC ATROPHY.**
VERFAHREN ZUR DIAGNOSE ODER VORHERSAGE EINER NICHT-SYNDROMISCHEN AUTOSOMALEN REZESSIVEN OPTISCHEN ATROPHIE ODER EINES RISIKOS EINER NICHT-SYNDROMISCHEN AUTOSOMALEN REZESSIVEN OPTISCHEN ATROPHIE
MÉTHODE DE DIAGNOSTIC OU DE PRÉDICTION D'UNE ATROPHIE OPTIQUE AUTOSOMIQUE RÉCESSIVE NON SYNDROMIQUE OU D'UN RISQUE D'UNE ATROPHIE OPTIQUE AUTOSOMIQUE RÉCESSIVE NON SYNDROMIQUE

(30) Priority: 24.03.2009 EP 09305259
(43) Date of publication of application: 01.02.2012
(73) Proprietor: INSERM - Institut National de la Santé et de la Recherche Médicale, 75013 Paris (FR); Université Paris Descartes, 75006 Paris (FR)
(72) Inventor: ROZET, Jean-Michel, F-75743 Paris Cedex 15 (FR); KAPLAN, Josseline, F-75743 Paris Cedex 15 (FR); PERRAULT, Isabelle, F-75743 Paris Cedex 15 (FR); GERBER, Sylvie, F-75743 Paris Cedex 15 (FR); HANEIN, Sylvain, F-75743 Paris Cedex 15 (FR); MUNNICH, Arnold, F-75743 Paris Cedex 15 (FR)
(74) Representative: Cabinet Plasseraud
(86) International application number: PCT/EP2010/053789
(87) International publication number: WO 2010/108926

(56) References cited:
- WO-A2-01/12659
- DATABASE EMBL -EBI [Online] 14 April 2003 (2003-04-14), "Expert Fasta Alignment Display" XP002531074 retrieved from EBI Database accession no. ABU52919
- STROM T M ET AL: "DIABETES INSIPIDUS, DIABETES MELLITUS, OPTIC ATROPHY AND DEAFNESS (DIDMOAD) CAUSED BY MUTATIONS IN A NOVEL GENE (WOLFRAMIN) CODING FOR A PREDICTED TRANSMEMBRANE PROTEIN" HUMAN MOLECULAR GENETICS, OXFORD UNIVERSITY PRESS, SURREY, vol. 7, no. 13, 1 January 1998 (1998-01-01), pages 2021-2028, XP002926970 ISSN: 0964-6906
- ZANNA C. ET AL.,: "OPAI mutations associated with dominanat optic atrophy impair oxidative phosphorylation and mitochondrial fusion" BRAIN, vol. 131, February 2008 (2008-02), pages 352-367, XP002531061
- HANEIN SYLVAIN ET AL: "TMEM126A, Encoding a Mitochondrial Protein, Is Mutated in Autosomal-Recessive Nonsyndromic Optic Atrophy" AMERICAN JOURNAL OF HUMAN GENETICS, vol. 84, no. 4, April 2009 (2009-04), pages 493-498, XP002583643 ISSN: 0002-9297
- ESTHER MEYER ET AL: 'Nonsense mutation in TMEM126A causing autosomal recessive optic atrophy and auditory neuropathy' MOLECULAR VISION, [Online] 13 April 2010, pages 650 - 664, XP055200243 Retrieved from the Internet: <URL:http://www.pubmedcentral.nih.gov/artic lerender.fcgi?artid=2855733&tool=pmcentrez& rendertype=abstract> [retrieved on 2015-07-06]

## Description

### FIELD OF THE INVENTION

The present invention relates to a method for diagnosing or predicting a non syndromic autosomal recessive optic atrophy, or a risk of a non syndromic autosomal recessive optic atrophy.

### BACKGROUD OF THE INVENTION

Hereditary optic atrophy is a group of neurodegenerative disorders characterized by a sudden or gradual loss of retinal ganglion cells function. The disease primarily involves the macular beam of the optic nerve and may progress to peripheral fibres. Leber hereditary optic neuropathy (LHON, [MIM 535000]) and autosomal dominant optic atrophies (adOA, [MIM 65500]) are by far the more frequent than recessive forms.
Typically, patients with LHON and adOA have no relevant health problems apart from optic atrophy. However, the disease may be associated with a wide range of extraocular features of mitochondrial dysfunction, including brain, cardiac, muscle or auditive signs. The clinical expression of extraocular manifestations varies from absence of expression to severe dysfunction defining the 'plus' forms of LHON and OPA1.¹⁻⁴

In contrast with LHON and adOA, autosomal recessive optic atrophies (arOA) usually involve the central nervous system and other organs. Non syndromic arOA are less common. Hitherto, one locus has been mapped but no disease gene has been identified (OPA6, *ROA1* [OMIM258500]).⁵

### SUMMARY OF THE INVENTION

The present invention relates to a method for diagnosing or predicting a non syndromic autosomal recessive optic atrophy, or a risk of a non syndromic autosomal recessive optic atrophy, in a subject, said method comprising detecting a mutation in the *TMEM126A* gene in a sample obtained from said subject, wherein the presence of a homozygous *TMEM126A* mutation is indicative of a non syndromic autosomal recessive optic atrophy or of a risk of a non syndromic autosomal recessive optic atrophy, wherein said TMEM126A mutation is a substitution of C by T at position 163 of SEQ ID NO: 1.

The inventors have discovered that defective TMEM126A is involved in the pathological process of non syndromic autosomal recessive optic atrophy. The present invention also relates to methods of screening for and detection of carriers of a defective *TMEM126A* gene, diagnosis of a defective *TMEM126A* gene, prenatal *TMEM126A* gene defect screening and detection.

### DETAILED DESCRIPTION OF THE INVENTION

### Definitions:

A "coding sequence" or a sequence "encoding" an expression product, such as a RNA, polypeptide, protein, or enzyme, is a nucleotide sequence that, when expressed, results in the production of that RNA, polypeptide, protein, or enzyme, i.e., the nucleotide sequence encodes an amino acid sequence for that polypeptide, protein or enzyme. A coding sequence for a protein may include a start codon (usually ATG) and a stop codon.

The term "gene" means a DNA sequence that codes for or corresponds to a particular sequence of amino acids which comprise all or part of one or more proteins or enzymes, and may or may not include regulatory DNA sequences, such as promoter sequences, which determine for example the conditions under which the gene is expressed. Some genes, which are not structural genes, may be transcribed from DNA to RNA, but are not translated into an amino acid sequence. Other genes may function as regulators of structural genes or as regulators of DNA transcription. In particular, the term gene may be intended for the genomic sequence encoding a protein, i.e. a sequence comprising regulator, promoter, intron and exon sequences.

As used herein, the term *"TMEM126A* gene" denotes the *TMEM126A* gene of any species, especially human, but also other mammals or vertebrates to which the methods of the invention can apply.
The human *TMEM126A* gene located at 11 q14.1, covers a genomic region of 8542 bp and is composed of 5 exons. The transcript is 773 bp long, with a coding sequence of 588 bp (exons 2-5, SEQ ID NO:1). The encoded protein TMEM126A (transmembrane protein 126A) is 195 amino-acids long (accession number NP_115649 (SEQ ID NO:2)).
We have chosen to number the A of the start codon (ATG) of the cDNA sequence of *TMEM126A* (Genbank accession numbers NM_ 032273) as nucleotide 1.
A nucleic acid molecule is "hybridizable" to another nucleic acid molecule, such as a cDNA, genomic DNA, or RNA, when a single stranded form of the nucleic acid molecule can anneal to the other nucleic acid molecule under the appropriate conditions of temperature and solution ionic strength (see Sambrook et al., 1989).
The conditions of temperature and ionic strength determine the "stringency" of the hybridization. For preliminary screening for homologous nucleic acids, low stringency hybridization conditions, corresponding to a Tm (melting temperature) of 55°C, can be used, e.g., 5x SSC, 0.1 % SDS, 0.25 % milk, and no formamide ; or 30 % formamide, 5x SSC, 0.5 % SDS). Moderate stringency hybridization conditions correspond to a higher Tm, e.g., 40 % formamide, with 5x or 6x SCC. High stringency hybridization conditions correspond to the highest Tm, e.g., 50 % formamide, 5x or 6x SCC. SCC is a 0.15 M NaCl, 0.015 M Nacitrate. Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher Tm) of nucleic acid hybridizations decreases in the following order: RNA:RNA, DNA:RNA, DNA:DNA. For hybrids of greater than 100 nucleotides in length, equations for calculating Tm have been derived (see Sambrook et al., 1989, 9.50-9.51). For hybridization with shorter nucleic acids, i.e., oligonucleotides, the position of mismatches becomes more important, and the length of the oligonucleotide determines its specificity (see Sambrook et al., 1989 11.7-11.8). A minimum length for a hybridizable nucleic acid is at least about 10 nucleotides, preferably at least about 15 nucleotides, and more preferably the length is at least about 20 nucleotides.
In a specific embodiment, the term "standard hybridization conditions" refers to a Tm of 55°C, and utilizes conditions as set forth above. In a preferred embodiment, the Tm is 60°C. In a more preferred embodiment, the Tm is 65°C. In a specific embodiment, "high stringency" refers to hybridization and/or washing conditions at 68°C in 0.2 X SSC, at 42°C in 50 % formamide, 4 X SSC, or under conditions that afford levels of hybridization equivalent to those observed under either of these two conditions.

As used herein, an amplification primer is an oligonucleotide for amplification of a target sequence by extension of the oligonucleotide after hybridization to the target sequence or by ligation of multiple oligonucleotides which are adjacent when hybridized to the target sequence. At least a portion of the amplification primer hybridizes to the target. This portion is referred to as the target binding sequence and it determines the target-specificity of the primer. In addition to the target binding sequence, certain amplification methods require specialized non-target binding sequences in the amplification primer. These specialized sequences are necessary for the amplification reaction to proceed and typically serve to append the specialized sequence to the target. For example, the amplification primers used in Strand Displacement Amplification (SDA) include a restriction endonuclease recognition site 5' to the target binding sequence (US Patent No. 5,455,166 and US Patent No. 5,270,184). Nucleic Acid Based Amplification (NASBA), self-sustaining sequence replication (3SR) and transcription based amplification primers require an RNA polymerase promoter linked to the target binding sequence of the primer. Linking such specialized sequences to a target binding sequence for use in a selected amplification reaction is routine in the art. In contrast, amplification methods such as PCR which do not require specialized sequences at the ends of the target, generally employ amplification primers consisting of only target binding sequence.
As used herein, the terms "primer" and "probe" refer to the function of the oligonucleotide. A primer is typically extended by polymerase or ligation following hybridization to the target but a probe typically is not. A hybridized oligonucleotide may function as a probe if it is used to capture or detect a target sequence, and the same oligonucleotide may function as a primer when it is employed as a target binding sequence in an amplification primer. It will therefore be appreciated that any of the target binding sequences disclosed herein for amplification, detection or quantisation of *TMEM126A* may be used either as hybridization probes or as target binding sequences in primers for detection or amplification, optionally linked to a specialized sequence required by the selected amplification reaction or to facilitate detection.

The terms "mutant" and "mutation" mean any detectable change in genetic material, e.g. DNA, RNA, cDNA, or any process, mechanism, or result of such a change. This includes gene mutations, in which the structure (e.g. DNA sequence) of a gene is altered, any gene or DNA arising from any mutation process, and any expression product (e.g. protein or enzyme) expressed by a modified gene or DNA sequence. Generally a mutation is identified in a subject by comparing the sequence of a nucleic acid or polypeptide expressed by said subject with the corresponding nucleic acid or polypeptide expressed in a control population. A mutation in the genetic material may also be "silent", i.e. the mutation does not result in an alteration of the amino acid sequence of the expression product.

In the context of the instant application, mutations identified in *TMEM126A* gene are designated pursuant to the nomenclature of Dunnen and Antonarakis (2000). As defined by Dunnen and Antonarakis at the nucleic acid level, substitutions are designated by ">", e.g. "163C>T" denotes that at nucleotide 163 of the reference sequence a C is changed to a T.

The expression"homozygous *TMEM126A* mutation", as used herein, refers to a subject whose two alleles of the *TMEM126A* gene are mutated. The expression thus encompasses both homozygous mutations *strico sensu* (wherein the same mutation is present on both alleles) and composite heterozygous mutation (wherein each allele presents a different mutation).

In the context of the invention, the term "treating" or "treatment", as used herein, means reversing, alleviating, inhibiting the progress of, or preventing the disorder or condition to which such term applies, or one or more symptoms of such disorder or condition. A "therapeutically effective amount" is intended for a minimal amount of active agent (e.g., *TMEM126A* encoding polynucleotide) which is necessary to impart therapeutic benefit to a subject. For example, a "therapeutically effective amount" to a mammal is such an amount which induces, ameliorates or otherwise causes an improvement in the pathological symptoms, disease progression or physiological conditions associated with or resistance to succumbing to a disorder.

The term "biological sample" means any biological sample derived from a subject. Examples of such samples include fluids, tissues, cell samples, organs, biopsies, etc. Preferred biological samples are a cell or tissue sample. Preferred biological samples are whole blood, serum, plasma or urine. Typically, in the case where the subject is a foetus, the sample may be an amniosynthesis sample.

As used herein, the term "subject" denotes a mammal, such as a rodent, a feline, a canine, and a primate. Preferably a subject according to the invention is a human.

### Diagnostic methods of the invention:

The inventors have shown that mutations found in the *TMEM126A* gene are associated with a non syndromic autosomal recessive optic atrophy. Defective TMEM126A is involved in the pathological process of non syndromic autosomal recessive optic atrophy.

Therefore, the present invention relates to a method for diagnosing or predicting a non syndromic autosomal recessive optic atrophy, or a risk of a non syndromic autosomal recessive optic atrophy, in a subject, said method comprising detecting a mutation in the *TMEM126A* gene in a sample obtained from said subject, wherein the presence of a homozygous *TMEM126A* mutation is indicative of a non syndromic autosomal recessive optic atrophy or of a risk of a non syndromic autosomal recessive optic atrophy, wherein said mutation is a substitution of C by T at position 163 of SEQ ID NO: 1.

The present invention also relates to a prenatal method for diagnosing or predicting a non syndromic autosomal recessive optic atrophy, or a risk of a non syndromic autosomal recessive optic atrophy, said method comprising detecting a mutation in the *TMEM126A* gene in a sample obtained from a foetus, wherein the presence of a homozygous *TMEM126A* mutation is indicative of a non syndromic autosomal recessive optic atrophy or of a risk of a non syndromic autosomal recessive optic atrophy , wherein said mutation is a substitution of C by T at position 163 of SEQ ID NO: 1.

The present disclosure also relates to a method for the detection of subject carrying a defective *TMEM126A* gene, which method comprises detecting a mutation in the *TMEM126A* gene in a sample obtained from said subject, wherein said mutation is a substitution of C by T at position 163 of SEQ ID NO: 1.

A *TMEM126A* mutation may be found in a regulating region of *TMEM126A* gene (e.g. a promoter sequence, or a binding site for transcription factor) or in exons that encode the TMEM126A protein.
Accoding to the disclosure, the *TMEM126A* mutation is a mutation which results in a reduction of *TMEM126A* expression or the *TMEM126A* mutation is a nonsense mutation which results in a truncated TMEM126A protein.

Themutation of *TMEM126A* gene according to the invention leads to a mutation of the arginine at residue 55 of the protein sequence, in particular mutation of *TMEM126A* gene according to the invention is 163C>T, i.e. a substitution of C by T at position 163 of SEQ ID NO:1.

In a preferred embodiment, the mutation is a homozygous mutation *stricto sensu,* wherein both alleles of the TMEM126A gene present the 163C<T substitution.
In another embodiment, the mutation is a composite heterozygous mutation, wherein one allele of the TMEM126A gene presents the 163C>T subsitution and the other allele presents another mutation.

Said mutation may be detected by analyzing a *TMEM126A* nucleic acid molecule. In the context of the invention, *TMEM126A* nucleic acid molecules include mRNA, genomic DNA and cDNA derived from mRNA. DNA or RNA can be single stranded or double stranded. These may be utilized for detection by amplification and/or hybridization with a probe, for instance.

The nucleic acid sample may be obtained from any cell source or tissue biopsy. Nonlimiting examples of cell sources available include without limitation blood cells, buccal cells, epithelial cells, fibroblasts, or any cells present in a tissue obtained by biopsy. Cells may also be obtained from body fluids, such as blood, plasma, serum, lymph, etc. DNA may be extracted using any methods known in the art, such as described in Sambrook et al., 1989. RNA may also be isolated, for instance from tissue biopsy, using standard methods well known to the one skilled in the art such as guanidium thiocyanate-phenol-chloroform extraction.

*TMEM126A* mutations may be detected in a RNA or DNA sample, preferably after amplification. For instance, the isolated RNA may be subjected to coupled reverse transcription and amplification, such as reverse transcription and amplification by polymerase chain reaction (RT-PCR), using specific oligonucleotide primers that are specific for the mutated site or that enable amplification of the region containing the mutated site. According to a first alternative, conditions for primer annealing may be chosen to ensure specific reverse transcription (where appropriate) and amplification; so that the appearance of an amplification product be a diagnostic of the presence of the particular *TMEM126A* mutation according to the invention. Otherwise, RNA may be reverse-transcribed and amplified, or DNA may be amplified, after which the mutated site may be detected in the amplified sequence by hybridization with a suitable probe or by direct sequencing, or any other appropriate method known in the art. For instance, a cDNA obtained from RNA may be cloned and sequenced to identify a mutation in *TMEM126A* sequence.

Actually numerous strategies for genotype analysis are available (Antonarakis et al., 1989; Cooper et al., 1991; Grompe, 1993). Briefly, the nucleic acid molecule may be tested for the presence or absence of a restriction site. When a base substitution mutation creates or abolishes the recognition site of a restriction enzyme, this allows a simple direct PCR test for the mutation. Further strategies include, but are not limited to, direct sequencing, restriction fragment length polymorphism (RFLP) analysis; hybridization with allele-specific oligonucleotides (ASO) that are short synthetic probes which hybridize only to a perfectly matched sequence under suitably stringent hybridization conditions; allele-specific PCR; PCR using mutagenic primers; ligase-PCR, HOT cleavage; denaturing gradient gel electrophoresis (DGGE), temperature denaturing gradient gel electrophoresis (TGGE), single-stranded conformational polymorphism (SSCP) and denaturing high performance liquid chromatography (Kuklin et al., 1997). Direct sequencing may be accomplished by any method, including without limitation chemical sequencing, using the Maxam-Gilbert method ; by enzymatic sequencing, using the Sanger method ; mass spectrometry sequencing ; sequencing using a chip-based technology; and real-time quantitative PCR. Preferably, DNA from a subject is first subjected to amplification by polymerase chain reaction (PCR) using specific amplification primers. However several other methods are available, allowing DNA to be studied independently of PCR, such as the rolling circle amplification (RCA), the InvaderTMassay, or oligonucleotide ligation assay (OLA). OLA may be used for revealing base substitution mutations. According to this method, two oligonucleotides are constructed that hybridize to adjacent sequences in the target nucleic acid, with the join sited at the position of the mutation. DNA ligase will covalently join the two oligonucleotides only if they are perfectly hybridized.

Therefore, useful nucleic acid molecules, in particular oligonucleotide probes or primers, according to the present invention include those which specifically hybridize the region where the mutation is located.

Oligonucleotide probes or primers may contain at least 10, 15, 20 or 30 nucleotides. Their length may be shorter than 400, 300, 200 or 100 nucleotides.

According to a further embodiment said mutation in the *TMEM126A* gene may be detected at the protein level.

Said mutation may be detected according to any appropriate method known in the art. In particular a sample, such as a tissue biopsy, obtained from a subject may be contacted with antibodies specific of the mutated form of TMEM126A, i.e. antibodies that are capable of distinguishing between a mutated form of TMEM126A and the wild-type protein (or any other protein), to determine the presence or absence of a TMEM126A specified by the antibody.

Antibodies that specifically recognize a mutated TMEM126A also make part of the disclosure. The antibodies are specific of mutated TMEM126A, that is to say they do not cross-react with the wild-type TMEM126A.

The antibodies of the present disclosure may be monoclonal or polyclonal antibodies, single chain or double chain, chimeric antibodies, humanized antibodies, or portions of an immunoglobulin molecule, including those portions known in the art as antigen binding fragments Fab, Fab', F(ab')2 and F(v). They can also be immunoconjugated, e.g. with a toxin, or labelled antibodies.

Whereas polyclonal antibodies may be used, monoclonal antibodies are preferred for they are more reproducible in the long run.

Procedures for raising "polyclonal antibodies" are also well known. Polyclonal antibodies can be obtained from serum of an animal immunized against the appropriate antigen, which may be produced by genetic engineering for example according to standard methods well-known by one skilled in the art. Typically, such antibodies can be raised by administering mutated TMEM126A subcutaneously to New Zealand white rabbits which have first been bled to obtain pre-immune serum. The antigens can be injected at a total volume of 100 µl per site at six different sites. Each injected material may contain adjuvants with or without pulverized acrylamide gel containing the protein or polypeptide after SDS-polyacrylamide gel electrophoresis. The rabbits are then bled two weeks after the first injection and periodically boosted with the same antigen three times every six weeks. A sample of serum is then collected 10 days after each boost. Polyclonal antibodies are then recovered from the serum by affinity chromatography using the corresponding antigen to capture the antibody. This and other procedures for raising polyclonal antibodies are disclosed by Harlow et al. (1988) which is hereby incorporated in the references.

A "monoclonal antibody" in its various grammatical forms refers to a population of antibody molecules that contains only one species of antibody combining site capable of immunoreacting with a particular epitope. A monoclonal antibody thus typically displays a single binding affinity for any epitope with which it immunoreacts. A monoclonal antibody may therefore contain an antibody molecule having a plurality of antibody combining sites, each immunospecific for a different epitope, e.g. a bispecific monoclonal antibody. Although historically a monoclonal antibody was produced by immortalization of a clonally pure immunoglobulin secreting cell line, a monoclonally pure population of antibody molecules can also be prepared by the methods of the present disclosure.

Laboratory methods for preparing monoclonal antibodies are well known in the art (see, for example, Harlow et al., 1988). Monoclonal antibodies (mAbs) may be prepared by immunizing purified mutated TMEM126A into a mammal, e.g. a mouse, rat, human and the like mammals. The antibody-producing cells in the immunized mammal are isolated and fused with myeloma or heteromyeloma cells to produce hybrid cells (hybridoma). The hybridoma cells producing the monoclonal antibodies are utilized as a source of the desired monoclonal antibody. This standard method of hybridoma culture is described in Kohler and Milstein (1975).

While mAbs can be produced by hybridoma culture the disclosureis not to be so limited. Also contemplated is the use of mAbs produced by an expressing nucleic acid cloned from a hybridoma of this disclosure. That is, the nucleic acid expressing the molecules secreted by a hybridoma of this disclosure can be transferred into another cell line to produce a transformant. The transformant is genotypically distinct from the original hybridoma but is also capable of producing antibody molecules of this disclosure, including immunologically active fragments of whole antibody molecules, corresponding to those secreted by the hybridoma. See, for example, U.S. Pat. No. 4,642,334 to Reading; PCT Publication No.; European Patent Publications No. 0239400 to Winter et al. and No. 0125023 to Cabilly et al.

Antibody generation techniques not involving immunisation are also disclosedsuch as for example using phage display technology to examine naive libraries (from non-immunised animals); see Barbas et al. (1992), and Waterhouse et al. (1993).

Antibodies raised against mutated TMEM126A may be cross reactive with wild-type TMEM126A. Accordingly a selection of antibodies specific for mutated TMEM126A is required. This may be achieved by depleting the pool of antibodies from those that are reactive with the wild-type TMEM126A, for instance by submitting the raised antibodies to an affinity chromatography against wild-type TMEM126A.

Alternatively, binding agents other than antibodies may be used. These may be for instance aptamers, which are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by Exponential enrichment (SELEX) of a random sequence library, as described in Tuerk C. and Gold L., 1990. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., 1999. Peptide aptamers consists of a conformationally constrained antibody variable region displayed by a platform protein, such as E. coli Thioredoxin A that are selected from combinatorial libraries by two hybrid methods (Colas et al., 1996).

Probe, primers, aptamers or antibodies of the disclosure may be labelled with a detectable molecule or substance, such as a fluorescent molecule, a radioactive molecule or any others labels known in the art. Labels are known in the art that generally provide (either directly or indirectly) a signal.

The term "labelled", with regard to the probe, primers, aptamers or antibodies of the disclosure, is intended to encompass direct labelling of the the probe, primers, aptamers or antibodies of the disclosure by coupling (i.e., physically linking) a detectable substance to the the probe, primers, aptamers or antibodies of the disclosure, as well as indirect labeling of the probe, primers, aptamers or antibodies of the disclosureby reactivity with another reagent that is directly labeled. Other examples of detectable substances include but are not limited to radioactive agents or a fluorophore (e.g. fluorescein isothiocyanate (FITC) or phycoerythrin (PE) or Indocyanine (Cy5)). Examples of indirect labeling include detection of a primary antibody using a fluorescently labeled secondary antibody and end-labeling of a DNA probe with biotin such that it can be detected with fluorescently labeled streptavidin. An antibody or aptamer of the disclosuremay be labelled with a radioactive molecule by any method known in the art. For example radioactive molecules include but are not limited radioactive atom for scintigraphic studies such as I123, I124, In111, Re186, Re188.

### Kits of the disclosure

According to another aspect of the disclosure, the *TMEM126A* mutation is detected by contacting the DNA of the subject with a nucleic acid probe, which is optionally labeled. Primers may also be useful to amplify or sequence the portion of the *TMEM126A* gene containing the mutated position of interest.

Such probes or primers are nucleic acids that are capable of specifically hybridizing with a portion of the *TMEM126A* gene sequence containing the mutated position of interest. That means that they are sequences that hybridize with the portion mutated *TMEM126A* nucleic acid sequence to which they relate under conditions of high stringency.
The present disclosure further provides kits suitable for determining at least one of the mutations of the *TMEM126A* gene.
The kits may include the following components:
(i) a probe, usually made of DNA, and that may be pre-labelled. Alternatively, the probe may be unlabelled and the ingredients for labelling may be included in the kit in separate containers; and
(ii) hybridization reagents : the kit may also contain other suitably packaged reagents and materials needed for the particular hybridization protocol, including solid-phase matrices, if applicable, and standards.
In another embodiment, the kits may include:
(i) sequence determination or amplification primers : sequencing primers may be pre-labelled or may contain an affinity purification or attachment moiety ; and
(ii) sequence determination or amplification reagents : the kit may also contain other suitably packaged reagents and materials needed for the particular sequencing amplification protocol. In one preferred embodiment, the kit comprises a panel of sequencing or amplification primers, whose sequences correspond to sequences adjacent to at least one of the polymorphic positions, as well as a means for detecting the presence of each polymorphic sequence.

In a particular embodiment, it is provided a kit which comprises a pair of nucleotide primers specific for amplifying all or part of the *TMEM126A* gene comprising at least one of mutations that are identified herein, especially position 163.

Alternatively, the kit of the disclosure may comprise a labelled compound or agent capable of detecting the mutated polypeptide of the disclosure (e.g., an antibody or aptamers as described above which binds the polypeptide). For example, the kit may comprise (1) a first antibody (e.g., attached to a solid support) which binds to a polypeptide comprising the mutation of the invention; and, optionally, (2) a second, different antibody which binds to either the polypeptide or the first antibody and is conjugated to a detectable agent.

The kit can also comprise, e.g., a buffering agent, a preservative, or a protein stabilizing agent. The kit can also comprise components necessary for detecting the detectable agent (e.g., an enzyme or a substrate). The kit can also contain a control sample or a series of control samples which can be assayed and compared to the test sample contained. Each component of the kit is usually enclosed within an individual container and all of the various containers are within a single package along with instructions for observing whether the tested subject is suffering from or is at risk of developing a non syndromic autosomal recessive optic atrophy.

### Therapeutic methods of the disclosure:

In a further object, the disclosure relates to use, methods and pharmaceutical compositions for treating or preventing a non syndromic autosomal recessive optic atrophy. Gene therapy is a particularly convenient way to treat a non syndromic autosomal recessive optic atrophy as it enables the provision of a constant supply of polypeptide or correction of the defective gene, for example as discussed below.

Gene therapy may be carried out by means of supplementation of cells lacking a functional TMEM126A polypeptide with a wild type *TMEM126A* gene product. Production of a suitable gene product may be achieved using recombinant techniques. For example, a suitable vector may be inserted into a host cell and expressed in that cell.

Thus the disclosure further relates to a method for treating or preventing a non syndromic autosomal recessive optic atrophy which comprises the step of administering a subject in need thereof with a *TMEM126A* polynucleotide, i.e. a nucleic acid sequence that encodes a wild-type *TMEM126A,* so that *TMEM126A* is expressed in vivo by the cells of the subject that have been transfected with said polynucleotide. Accordingly, said method leads to an overexpression of wild-type *TMEM126A* which compensates expression of defective mutated *TMEM126A.*

The disclosure also relates to the use of a *TMEM126A* polynucleotide for the manufacture of medicament intended for the treatment of a non syndromic autosomal recessive optic atrophy.

Said *TMEM126A* polynucleotide is administered in a therapeutically effective amount.

Preferably the *TMEM126A* polynucleotide sequence according to the disclosure is associated with elements that enable for regulation of its expression, such as a promoter sequence.

Such a nucleic acid may be in the form of a vector. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors, expression vectors, are capable of directing the expression of genes to which they are operably linked. In general, expression vectors of utility in recombinant DNA techniques are often in the form of plasmids (vectors). However, the disclosureis intended to include such other forms of expression vectors, such as viral vectors (e.g., replication defective retroviruses, adenoviruses and adeno-associated viruses (AAV)), which serve equivalent functions.
In a preferred embodiment, the expression vector is an AAV vector.
Adeno-associated viral (AAV) vectors have become powerful gene delivery tools for the treatment of retinal degeneration. AAV vectors possess a number of features that render them ideally suited for retinal gene therapy, including a lack of pathogenicity, minimal immunogenicity, and the ability to transduce postmitotic cells in a stable and efficient manner. In the sheltered environment of the retina, AAV vectors are able to maintain high levels of transgene expression in the retinal pigmented epithelium (RPE), photoreceptors, or ganglion cells for long periods of time after a single treatment. Each cell type can be specifically targeted by choosing the appropriate combination of AAV serotype, promoter, and intraocular injection site (Dinculescu et al., Hum Gene Ther. 2005 Jun;16(6):649-63).

The *TMEM126A* polynucleotide may be introduced into a target cell by means of any procedure known for the delivery of nucleic acids to the nucleus of cells, ex vivo, on cells in culture or removed from an animal or a patient, or in vivo.

Ex vivo introduction may be performed by any standard method well known by one skilled in the art, e.g. transfection, electroporation, microinjection, transduction, cell fusion, DEAE dextran, calcium phosphate precipitation, or use of a gene gun.

The *TMEM126A* polynucleotide can also be introduced ex vivo or in vivo by lipofection. In certain embodiments, the use of liposomes and/or nanoparticles is contemplated for the introduction of the donor nucleic acid targeting system into host cells.

Nanocapsules can generally entrap compounds in a stable and reproducible way. To avoid side effects due to intracellular polymeric overloading, such ultrafine particles (sized around 0.1 µm) should be designed using polymers able to be degraded in vivo. Biodegradable polyalkyl-cyanoacrylate nanoparticles that meet these requirements are contemplated for use in the present disclosure, and such particles may be are easily made.

Liposomes are formed from phospholipids that are dispersed in an aqueous medium and spontaneously form multilamellar concentric bilayer vesicles (also termed multilamellar vesicles (MLVs)). MLVs generally have diameters of from 25 nm to 4 µm. Sonication of MLVs results in the formation of small unilamellar vesicles (SUVs) with diameters in the range of 200 to 500 Å, containing an aqueous solution in the core.

Synthetic cationic lipids designed to limit the difficulties and dangers encountered with liposome mediated transfection can be used to prepare liposomes for in vivo transfection of a gene encoding a marker. The use of cationic lipids may promote encapsulation of negatively charged nucleic acids, and also promote fusion with negatively charged cell membranes (Felgner et al., 1989).

Alternatively, one of the simplest and the safest way to deliver *TMEM126A* polynucleotide across cell membranes in vivo may involve the direct application of high concentration free or naked polynucleotides (typically mRNA or DNA). By "naked DNA (or RNA)" is meant a DNA (RNA) molecule which has not been previously complexed with other chemical moieties. Naked DNA uptake by animal cells may be increased by administering the cells simultaneously with excipients and the nucleic acid. Such excipients are reagents that enhance or increase penetration of the DNA across cellular membranes and thus delivery to the cells delivery of the therapeutic agent. Various excipients have been described in the art, such as surfactants, e.g. a surfactant selected form the group consisting of Triton X-100, sodium dodecyl sulfate, Tween 20, and Tween 80; bacterial toxins, for instance streptolysin O, cholera toxin, and recombinant modified labile toxin of E coli; and polysaccharides, such as glucose, sucrose, fructose, or maltose, for instance, which act by disrupting the osmotic pressure in the vicinity of the cell membrane. Other methods have been described to enhance delivery of free polynucleotides, such as blocking of polynucleotide inactivation via endo- or exonucleolytic cleavage by both extra- and intracellular nucleases.

Alternatively, the disclosure also provides a method for treating or preventing a non syndromic autosomal recessive optic atrophy which comprises the step of administering a subject in need thereof with a wild-type TMEM126A.

Knowing the amino acid sequence of the desired sequence, one skilled in the art can readily produce said polypeptides, by standard techniques for production of polypeptides. For instance, they can be synthesized using well-known solid phase method, preferably using a commercially available peptide synthesis apparatus (such as that made by Applied Biosystems, Foster City, California) and following the manufacturer's instructions.

Alternatively, the polypeptides of the disclosure can be synthesized by recombinant DNA techniques as is now well-known in the art. For example, these fragments can be obtained as DNA expression products after incorporation of DNA sequences encoding the desired (poly)peptide into expression vectors and introduction of such vectors into suitable eukaryotic or prokaryotic hosts that will express the desired polypeptide, from which they can be later isolated using well-known techniques.

Polypeptides of the disclosurecan be use in an isolated (e.g., purified) form or contained in a vector, such as a membrane or lipid vesicle (e.g. a liposome).

### Transgenic non-human animal models of non syndromic autosomal recessive optic atrophy

The present invention further relates to an in vivo model of non syndromic autosomal recessive optic atrophy: a transgenic non-human animal which is TMEM126A-deficient due to a mutation in the *TMEM126A* gene,wherein said *TMEM126A* mutation is a substitution of C by T at position 163 of SEQ ID NO: 1.
By transgenic non-human animal which is TMEM126A-deficient, it is meant a homozygous animal (i.e., the two alleles of *TMEM126A* are defective).
Typically suitable transgenic non-human animals are laboratory animals such as rodents, rats, mice, monkeys, dogs, rabbits, guinea pigs, goats, sheep, pigs and cattle. In one embodiment the transgenic non-human animal of the present invention is a rodent. In a more preferred embodiment the transgenic animal is a mouse.
Typically the transgenic animals according to the invention are generated using General Methods Standard molecular biology techniques known in the art (see for example US Patent 6740793, US2006/0101533).

The disclosure provides a means for identification of agents that interfere, delay or inhibit processes involved in diseases or conditions involving a mutated *TMEM126A* gene, such as non syndromic autosomal recessive optic atrophy. Such agents would be of significant clinical importance for treatment of non syndromic autosomal recessive optic atrophy. The provision of the animal model according to the present invention can greatly shorten the time required for screening for such agents. Thus the present disclosure relates to a method for identifying a compound useful for treating, preventing or inhibiting non syndromic autosomal recessive optic atrophy, comprising the step of contacting a candidate compound with a transgenic animal according to the invention.

The invention will further be illustrated in view of the following figures and example.

### FIGURES

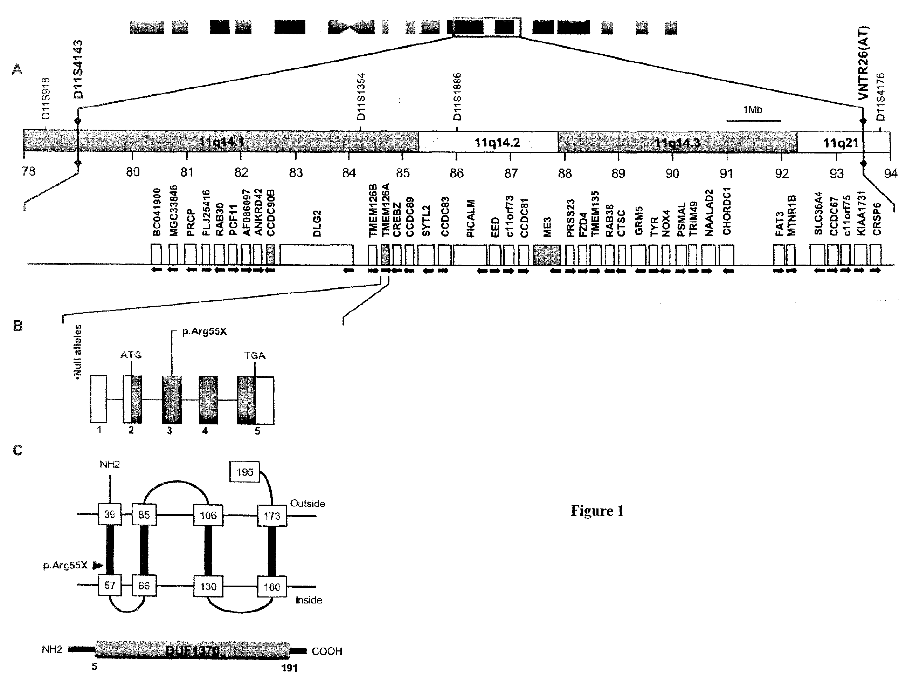

**Figure 1****: Critical region of *ROA2* locus, structure of *TMEM126A* gene and mutation identified in four arOA families.**
   **(A)** Physical map of human chromosome 11q14.1-q21 with selected genetic markers. Location and transcriptional direction of known genes are schematically represented and distances are indicated relative to chromosome 11 according to the Ensembl and UCSC Genome Browser databases. Mitochondrial candidate genes tested in our study are in grey: Coiled-coil domain containing (*CCDC90B;* Maestro score⁷ = +15), transmembrane protein 126A (*TMEM126;* Maestro score = +16) and malic enzyme 3 (*ME3*; Maestro score = +23).
   **(B)** Structure of the *TMEM126A* gene and position of the disease causing mutation identified in arOA families. The gene, located at 11 q14.1, covers a genomic region of 8542 bp and is composed of 5 exons. The transcript is 773 bp long, with coding sequence of 585 bp (exons 2-5, NM_032273.2). The coding region is indicated in grey and UTRs (5' and 3') in white. **(C)** The transmembrane 126A protein is predicted to contain four transmembrane domains (PredictProtein) and a domain of unknown function DUF1370 (Pfam). Chromatograms of *TMEM126A* genomic and cDNA sequences have shown a homozygous nonsense c.163C>T; p.Arg55X identified in patient III-6 family 1. The arginine at residue 55 is highly conserved in different species (NCBI BLAST).

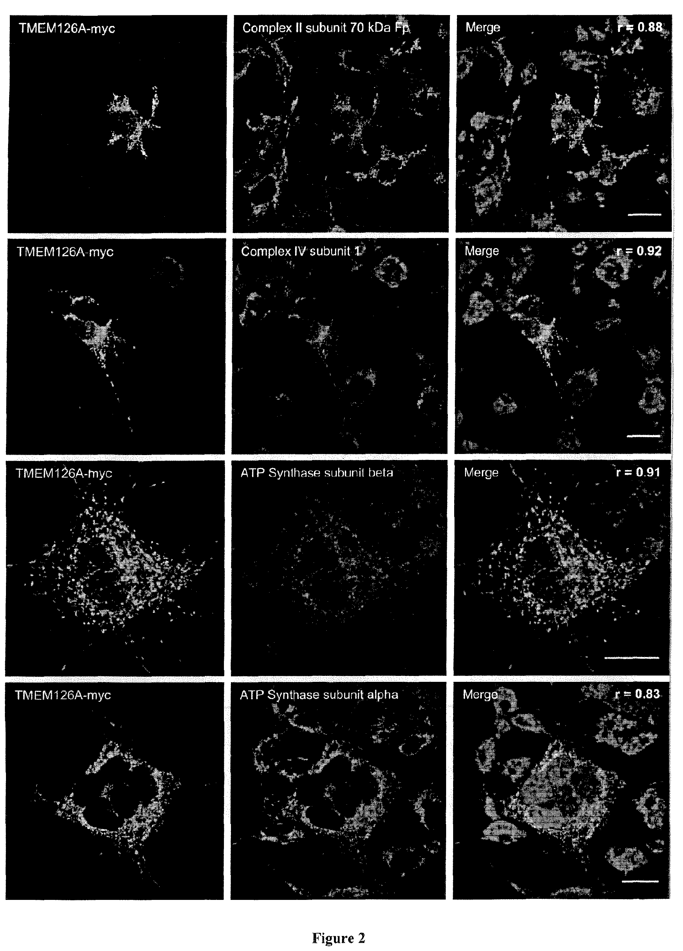

**Figure 2****: Localisation of transmembrane 126A protein to mitochondria of cultured cells.** Expression in COS-7 cells of a TMEM126-myc fusion protein labeled 48 hours after transfection with an antibody against the myc tag (green) compared to mitochondrial markers (red) using the following primary antibodies: mouse monoclonal anti-Complex II subunit 70 kDa Fp (1/2000, MitoSciences), mouse monoclonal anti-Complex IV subunit 1 (1/200, MitoSciences), mouse monoclonal anti-ATP Synthase subunit beta (1/400, MitoSciences) and mouse monoclonal anti-ATP Synthase subunit (1/500, MitoSciences): Complex II subunit 70 kDa Fp, Complex IV subunit 1, ATP Synthase subunit beta and ATP Synthase subunit alpha, respectively. The Pearson's coefficient (r) was calculated using the ImageJ 1.42d software to estimate the degree of colocalisation between TMEM126-myc and organelle markers. Its value can range from 1 to -1, with 1 standing for complete positive correlation and -1 for a negative correlation, with zero standing for no correlation. Pearson's coefficients between TMEM126-myc and mitochondria markers are > 0.83 showing a strong colocalization. These results were confirmed by JACoP Van Steenel's CCF coefficients²¹ and Pearson's coefficients calculated with Imaris 6.1.5 software (data not shown). Images were acquired using a Leica SP5 confocal microscope (objective x63, scale bar = 20 µm) and ***Leica Application Suite Advanced Fluorescence Lite software.***

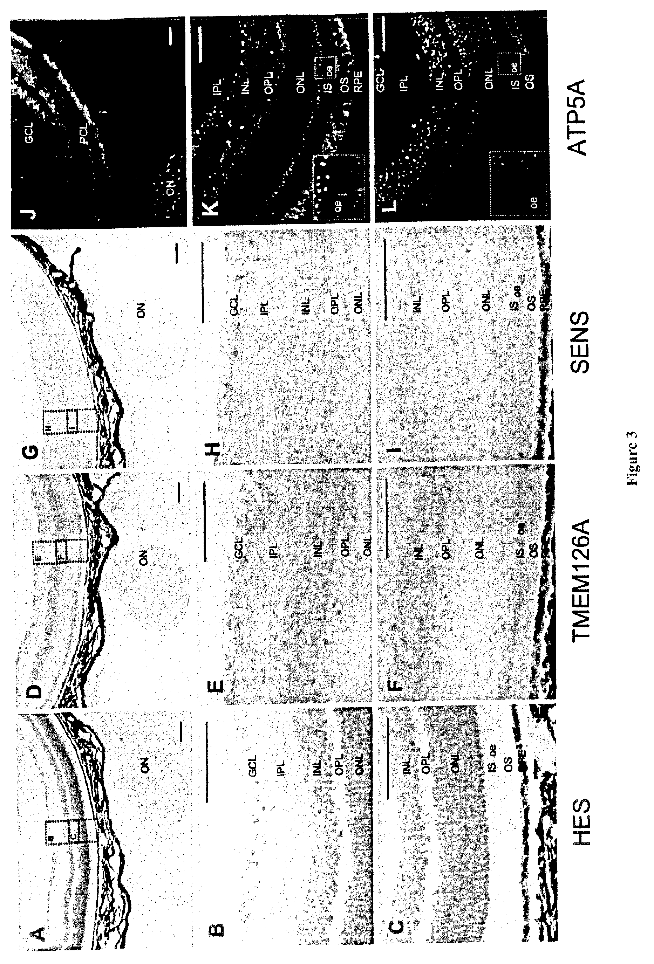

***Figure 3******: Analyse of TMEM126 mRNA expression by in situ hybridization compared to* localization of mitochondria in adult mouse retina.** Murine retina cDNA was amplified using primers 5'-T7-acagagtcacgtgtgagccaa-3' and 5'-ccattgacgggtatagccaa-3' (sens probe) and 5'-T7-ccattgacgggtatagccaa-3' and 5'-acagagtcacgtgtgagccaa-3' (anti-sens probe) to produce a 524-bp product of the mouse Tmem126a coding region (NM_025460, primer T7: 5'-taatacgactcactatagggaga-3'). The PCR products were purified (QIAquick PCR Purification Kit), verified by sequencing and subsequently amplified by PCR using T7-polymerase specific primers (Invitrogen) and Digoxigenin (DIG) RNA labeling mix (Roche). The DIG-labeled cRNA probes were hybridized to the mouse retina sections and detected using an anti-digoxygenin alkaline phosphatase-linked antibody (Roche) and visualized using NBT/BCIP (Roche). (**A-L**) Magnification of the mouse retinal cell layers. **(D-L)** In adult mouse retina *TMEM126* gene is expressed in ganglion cell layer (GCL), in the inner nuclear layer (INL), outer plexiform layer (OPL) and the outer ellipsoide (oe) length of photoreceptors inner segments (IS). The staining is slightly less pronounced in the optic nerve (ON) and in the inner plexiform layer. *TMEM126A* mRNA was not detected in the outer nuclear layer (ONL), outer plexiform layer and in the inner plexiform layer. **(G-I)** A sense DIG-cRNA probe revealed no staining in all cellular layers indicating the specificity of the assay. **(J, K)** Immunolocalization of mitochondria in mouse retinal cell layers with an anti-ATP5A. Mitochondria are sequestered in ganglion cell layer, outer plexiform layer, outer ellipsoid length of the photoreceptors inner segments, retinal pigment epithelium cells and in the optic nerve. **(L)** Negative control. Note that outer segments display an auto-fluorescence. PCL, photoreceptor cell layer; OS, outer segments; RPE, retinal pigment epithelium. Images were recorded on an Olympus IX81 microscope (objective 10x and 40x, scale bar = 100 µm) with an Olympus DP70 camera, processed with Soft Imaging System Cell^P and Leica SP5 confocal microscope (objective 40x oil immersion objective, scale bar = 50 µm) and Leica Application Suite Advanced Fluorescence Lite software for immunohistochemistry.

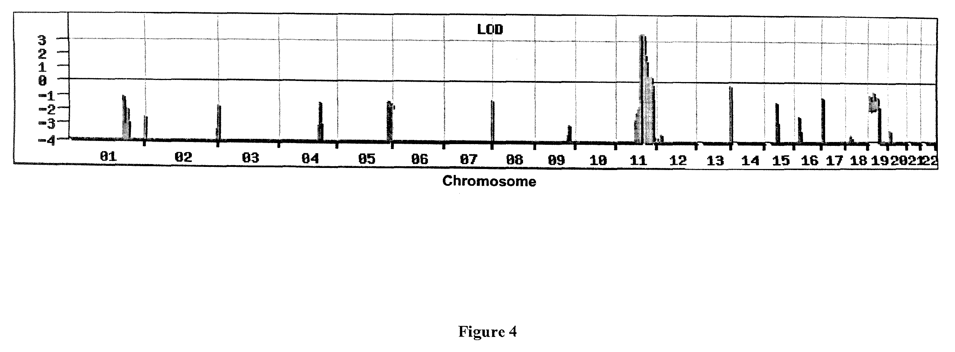

**Figure 4****.** Full parametric linkage analysis of Family 1 using a combination of Affymetrix GeneChip Human Mapping 10K 2.0 Arrays and microsatellite markers. This approach points to a unique candidate region on chromosome 11 with a maximum lod-score >+3. Parametric LOD scores were calculated using the MERLIN22 software program.

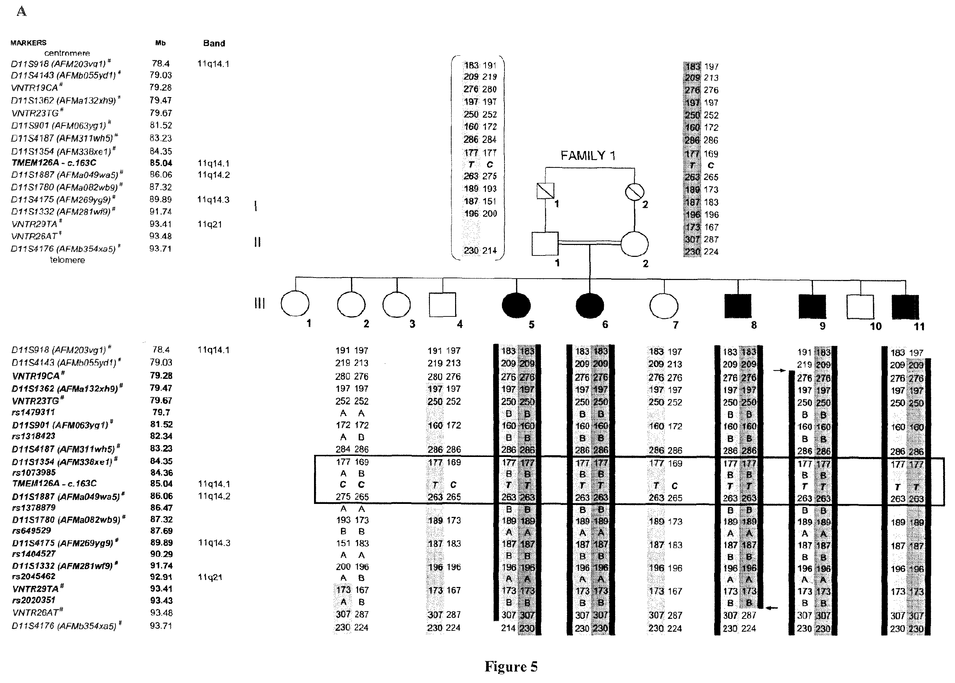

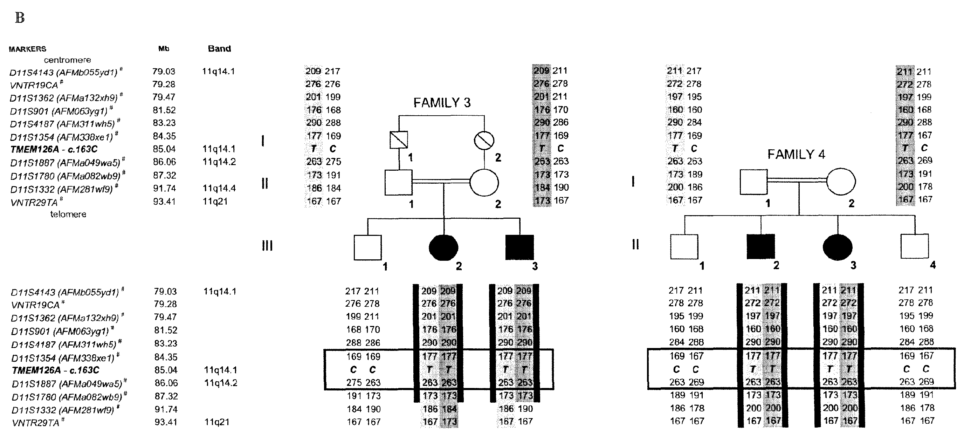

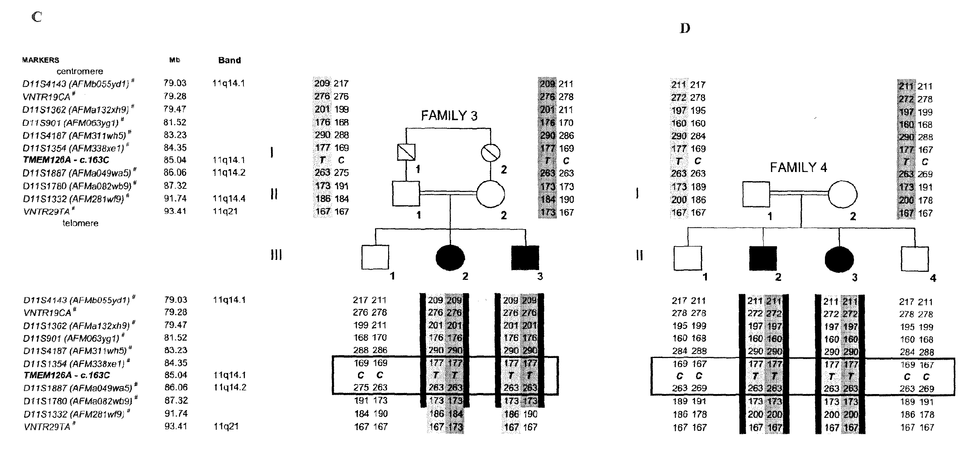

**Figure 5****. Localization of the ROA2 locus and segregation of disease-causing mutations.**
   Pedigree structure of families 1 to 4 with haplotypes reconstruction for informative markers on chromosome 11q14.1-q21. Black circles (women) and squares (men) indicate affected members. The code numbers of all sampled individuals are given below the symbols. Chromosomal positions of SNP and microsatellite markers are indicated in Mega base pairs (Mb) according to the draft of the human genome sequence (UCSC and Ensembl databases). VNTR: variable number of tandem repeat chosen from the UCSC database (primers in Table 1). Brackets show the deduced haplotype of subject II-1. Hashes point multiallelic markers used to refine the interval indicated by the "Affimetrix 10k SNP" genome scan in individuals III-2, III-5, III-6, III-8 and III-9 (Family 1). The homozygous haplotype in which the mutated gene is most likely located in affected patients is flanked by black boxes. Arrows indicate the position of key recombination events that were used to restrict the candidate ROA2 interval: obligatory recombination events between loci D11S4143 and VNTR19CA (Family 1, patient III-9), and loci rs2020351 and VNTR26AT (family 1, individual III-8), respectively, defined a 14.4 Mb interval between loci D11S4143 and VNTR26(AT). The ancestral haplotype shared by the four families is framed. Segregation of disease-causing mutations: "C" indicates wild-type allele and "T" indicates the nonsense mutation c.163C>T, p.Arg55X. Mutations were detected after PCR of the five TMEM126A exons (primers in Table 1), sequencing of the purified fragments using the Big Dye chemistry version 3.1 (Applied Biosystems) and analysis using an ABI-3130 sequencer (Applied Biosystems). We have chosen to number the A of the start codon (ATG) of the cDNA sequence of the *TMEM126A* (Genbank accession numbers NM_ 032273) as nucleotide 1.

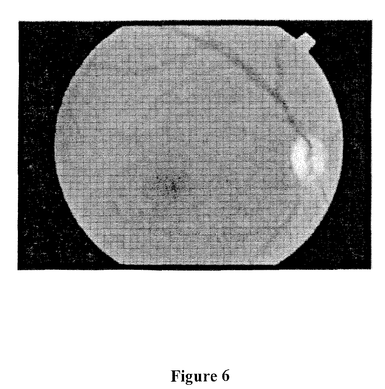

**Figure 6****. Ophthalmological data in 37-year-old patient III6, Family 1 harboring the TMEM126 p.Arg55X mutation.**
   The fundus photograph of left eye shows optic disc pallor with normal aspect of the retina including the macular region. Flash visual evoked potentials (VEP) showed flattened waves, prolonged latencies and highly reduced amplitudes for right eye (RE) and left eye (LE). Electroretinographic recordings were strictly normal. Pattern reversal VEP displayed hardly recordable traces. Goldman Dynamic perimetry show severe bilateral visual field loss.

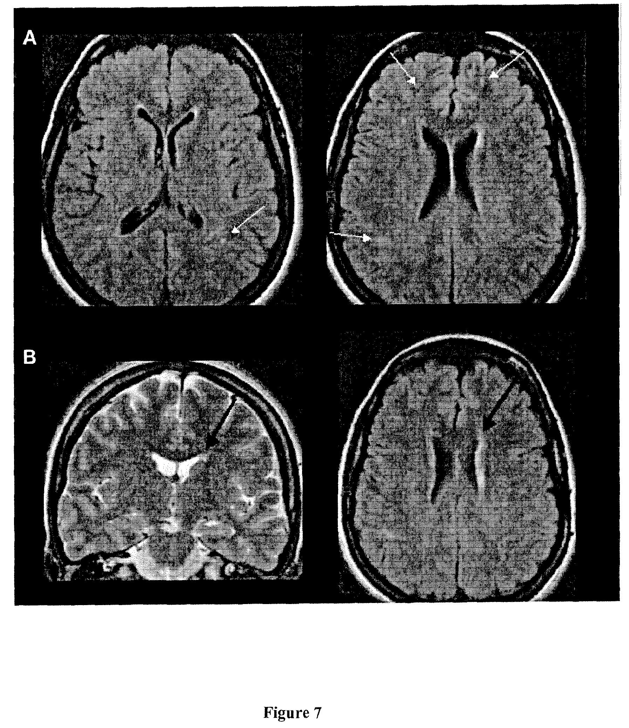

**Figure 7****. Brain Magnetic Resonance Imaging (MRI) of 37-year-old patient III6, Family 1 harboring the TMEM126 p.Arg55X mutation.**
   **(A)** Axial FLAIR weighted images show asymmetric and homogeneous punctate hyperintensities with no evidence for necrosis (white arrows). **(B)** Coronal weighted FSE T2 and axial FLAIR images show hyperintensities in the bilateral stratum subependymale (left > right side) near the head and corpus of the caudate nuclei (black arrow).

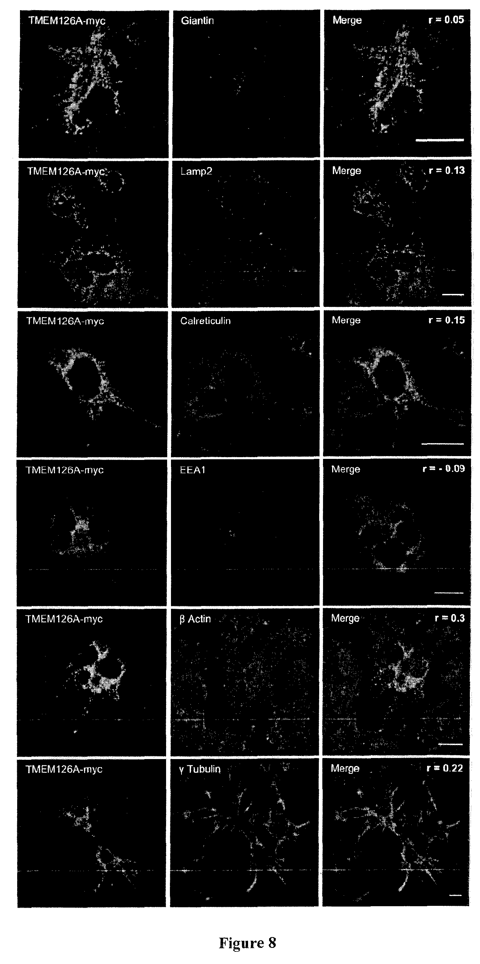

**Figure 8****. Expression of the TMEM126-myc fusion protein in COS-7 cells, 48 hours after transient transfection.**
   COS-7 cells were transfected with 1 µg plasmid DNA (OmicsLink ORF Expression Clone-GeneCopoeiaTM clone EX-V0975-M09; Ressourcenzentrumfuer Genomforschung imaGenes) with FuGENE®6 transfection reagent, according to the manufacturer's instructions (Roche). The cells were fixed for 15 min in 4% formaldehyde, 48 hr posttransfection. Immunocytochemistry was performed via classical procedures. Colocalization experiments were performed using specific or myc antibodies (mouse monoclonal anti-myc and rabbit polyclonal anti-myc, 1/200, 0.2 mg/mL, Santa Cruz Biotechnology), respectively. Secondary antibodies were Alexa Fluor®488 conjugated goat anti-rabbit (1/1000, Molecular Probes) and ZyMax™ goat anti-mouse CY™ 3 conjugate (1/100, Invitrogen). No significant colocalization of Transmembrane 126A protein was noted with Golgi apparatus (mouse monoclonal anti-Giantin, 1/200, Abcam), lysosomes (mouse monoclonal anti-Lamp2, 1/200, Abcam), endoplasmic-reticulum (mouse monoclonal anti-Calreticulin, 1/400, Stressgen), early endosomes (rabbit polyclonal anti-EEA1, 1/200, Abcam), cytoskeleton (mouse monoclonal anti-ßActin, 1/100, AbCys) and microtubule (mouse monoclonal anti-.Tubulin, 1/200, Sigma), respectively. Cells were counterstained and mounted with ProLong®Gold antifade reagent with DAPI (Invitrogen). Images were recorded using a Leica SP5 confocal microscope (objective 63x oil immersion objective, scale bar = 20 µm) and Leica Application Suite Advanced Fluorescence Lite software. Expression of a myctagged fusion protein of the expected size was verified on Western blots of cell extracts with a myc antibody (mouse anti myc, 1/1000, Santa Cruz Biotechnology, data not shown).

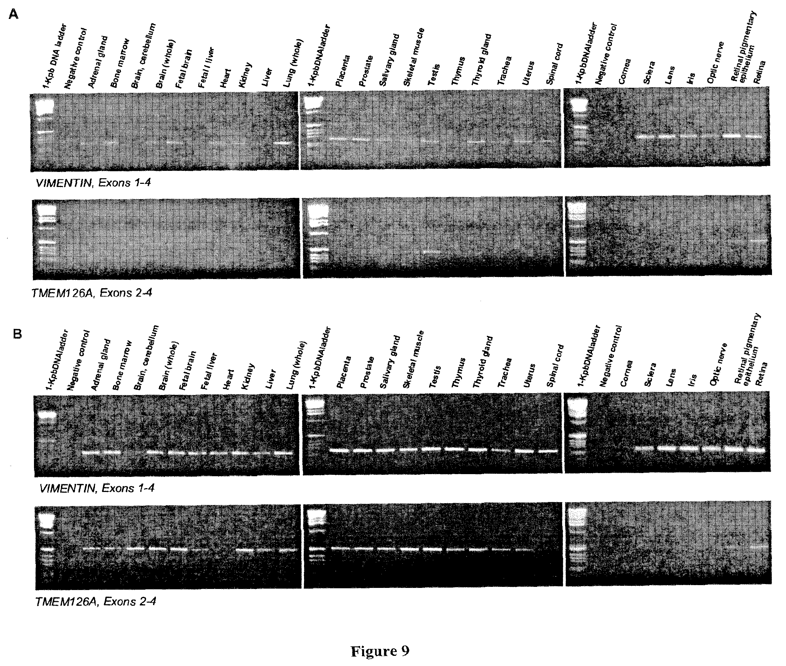

**Figure 9****. RT-PCR quantification of TMEM126A mRNA in human tissues.**
   *TMEM126A* mRNA expression was analyzed by RT-PCR in adult human tissues, normalized with respect to vimentin expression, **(A)** semiquantitatively (at 25 PCR cycles, in the exponential phase) and **(B)** at end point PCR (at 35 cycles, in the plateau phase). One microgram of total RNAs from adrenal gland, bone marrow, brain, cerebellum, brain fetal brain, fetal liver, lung (whole), placenta, prostate, salivary gland, skeletal muscle, testis, thymus, thyroid gland, trachea, uterus and spinal cord (Human Total RNA Master panel II, Clontech) were reverse transcribed with the High Capacity cDNA Archive Kit (Applied Biosystems) primed with Oligo d(T)16 (Applied Biosystems) in accordance with the supplier's recommendations. 0.5 microgram of total RNAs from retina, optic nerve, retinal pigmentary epithelium (RPE), cornea, iris, sclera and lens extracted from a twenty-week-old fetal human ocular globe were also reverse transcribed. A 523 pb cDNA fragment of *TMEM126A* from exon 2 to exon 5 was amplified using primer: (forward) 5'-taaccagcttccagaagcag-3', (reverse) 5'-gtgaatttctttgccaggttca-3' (Genbank accession numbers NM_032273). A 274 bp cDNA fragment of VIMENTIN containing exons 1 to 4 was amplified using primers: (forward) 5'accagctaaccaacgacaaa-3', (reverse) 5'-tgctgttcctgaatctgagc-3' (GenBank accession number NM_003380), as a reference. Semi-quantitative condition showed a predominantly expression in brain (whole), cerebellum, fetal brain, skeletal muscle, testis, fetal retinal pigmentary epithelium (RPE) and retina **(A)** while a widespread expression of *TMEM126A* mRNA in all adult tissues was noted **(B).**

**Table 1: Sequences of primers used for amplification of (A) four polymorphic markers developed from the UCSC Draft of the Human Sequence; and (B) the five exons of the TMEM126 gene (Genbank accession number NM_032273).**

| **A) Polymorphic markers** (VNTR = variable number of tandem repeat). | | | |
|---|---|---|---|
| VNTRs / chromosomal position | | Forward sequence (5'-3') | Reverse sequence (5'-3') |
| VNTR19CA (79.28Mb) | | tgccgggagcctaaaat | actcaccctgcagatcttag |
| VNTR23TG (79.67Mb) | | gatacatgtatacattgtgt | gttaatcattttgacatgtt |
| VNTR29TA (93.41 Mb) | | tcactcaactcctgaacctc | aagatctaccccagtgcg |
| VNTR26AT (93.48Mb) | | gcttcctcattccctctctg | tagcctattgtgggaccc |
| | | | |

| **B) TMEM126** | | | |
|---|---|---|---|
| Exon | Forward sequence (5'-3') | Reverse sequence (5'-3') | |
| 1 | cttctcagcccaaagccgct | cgtcgtgcttctcctgacac | |
| 2 | cagattagctctacagtattat | ccttctcacattccatgttg | |
| 3 | gggatagatgtcgtatccag | attacagcatacagtacttgg | |
| 4 | caattacatttgatatattacctg | gggtaacattgcctttggtc | |
| 5 | tttatcttaagacttctaggac | ccttttgttgtaggtcccag | |

### EXAMPLE

### Abstract

Non-syndromic autosomal recessive optic neuropathies are rare conditions of unknown genetic and molecular origin. Using a whole-genome homozygosity mapping and positional cloning approach, we have identified the first gene, to our knowledge, responsible for this condition, *TMEM126A,* in a large multiplex inbred Algerian family and subsequently in three other families originating from Maghreb. *TMEM126A* is conserved in higher eukaryotes and encodes a transmembrane mitochondrial protein supporting to the view that mitochondrial dysfunction may be a hallmark of inherited optic neuropathies including isolated autosomal recessive forms.

Using a combination of Affymetrix GeneChip Human Mapping 10K 2.0 Arrays and microsatellite markers, we performed homozygosity mapping in a multiplex inbred non-syndromic arOA family of Algerian ancestry. This approach identified a unique region of homozygosity on chromosome 11q14.1-q21 (*ROA2,* Family 1, Figures 4 and 5). The critical interval spanned 14.4 Mb and contained 40 known genes (Figure 1; maximum lod-score Zmax = 3.73 with no recombination event at the *D11S4187* locus).
We selected in the interval three genes predicted to encode mitochondrial products⁷ (Figure 1). Systematic sequence analysis identified a homozygous nonsense mutation in the gene encoding *TMEM126A,* a transmembrane protein (c.163C>T; p.Arg55X; Genbank accession NM_032273; Figure 1; Figure 5). The mutation occurred in a CpG doublet, segregated with the disease in the family (Figure 5) and was not found in 700 control chromosomes (European n= 600; Algerian n=100).
We screened for *TMEM126A* mutations 48 additional unrelated patients affected with non-syndromic OA (arOA n=14; adOA n=10; sporadic n=24). All Affected patients and their relatives, including all members of Family 1, were recruited with their informed and written consent, as prescribed by the law on bioethics of the European Community and after approval by the local ethics committee (DC-2008-512, Paris-Necker). In all cases, OPA1 mutations and the most frequent LHON mutations (mtDNA G11778A, G3460A, T14484C and G15257, respectively) were previously excluded by direct sequencing. None of the sporadic and adOA cases carried *TMEM126A* mutations. Conversely, the p.Arg55X mutation was identified in three additional arOA families of Maghrebian origin (Tunisa, Family 2; Morocco, Families 3 and 4; Figure 5).
Segregation analysis of microsatellite markers flanking the mutation supported the hypothesis of a founder effect by showing the transmission of a small common haplotype with the disease in the four families (Figure 5). Haplotype studies as well as Bayesian calculations⁸ suggested that the *TMEM126A* c.163C>T mutation occurred 80 generations ago, *i.e.* about 2400 years ago (95% credible interval 35-150 generations).
The p.Arg55X mutation caused an early-onset severe bilateral deficiency in visual acuity (VA), optic disc pallor and central scotoma (onset between 4 to 6 years; VA = counting fingers to 20/200; Figure 6). The peripheral visual field was strictly normal in all but the oldest patient who lost it between the age of 30 and 37 (III6, Family 1; Figure 6).
The phenotype differs from that of *ROA1* patients (OPA6 [MIM258500]) presenting with an early onset but slowly progressive OA affecting the macular beam of optic nerve with preservation of the peripheral bundles in advanced stage.⁵
Polarographic tests and spectrophotometric assays on cultured skin fibroblasts⁹ showed normal respiratory chain function in Patient III6 - Family 1 but partial deficiency of complex I in Patient V1 - Family 2 (17 nmol/min/mg of protein, mean = 37 +/-MSD 5 nmol/min/mg of protein). Patient V1 - Family 2 presented with normal brain MRI but moderate hypertrophic cardiomyopathy. These features along with the minor brain MRI alterations (Figure 7) and mild hearing loss in Patient III6 - Family 1 are suggestive of a mitochondrial dysfunction as previously noted in patients with LHON or OPA1 mutations.^{1-4, 10}

The *TMEM126A* gene spans 8.5 kb on chromosome 11 and encodes a single ubiquitous transcript (770 bp) made of one non-coding and four coding exons (Figure 1).
The TMEM126 protein includes the Pfam domain of unknown function defined by the homology between the TMEM126A p.Lys5-Gly191 and TMEM126B p.Asn45-229Glu aminoacid sequences, respectively. Four transmembrane domains with the N terminal and C terminal sequences on the outside of the membrane were predicted in the TMEM126A DUF1370 domain (PredictProtein, Figure 1). DUF proteins are hypothetical eukaryotic proteins of around 200 residues in length. Members of this family are regarded as specific to mammals but phylogenetic analyses contradict this notion. Indeed, orthologs of several DUF proteins, including TMEM126A, have been found in many vertebrate and invertebrate species as well as in plant organisms.^{11, 12} No ortholog could be identified in yeast and fungi. To determine its subcellular localization we overexpressed a TMEM126A-myc fusion protein into COS-7 cells. Epitope-tagged wild-type TMEM126A colocalized with mitochondrial Complex II subunit 70 kDa Fp (SDHA [MIM 600857]), Complex IV subunit 1 (MTCO1 [MIM 516030]), ATP Synthase subunit beta (ATP5B [MIM 102910]) and ATP-Synthase subunit alpha (ATP5A [MIM 164360]) supporting the mitochondrial localization of the protein (Figure 2). The fusion protein did not significantly colocalize with the markers of the Golgi apparatus, lysosomes, early endosomes, cytoskeleton and microtubule - centrosome, respectively (Figure 8).
RT-PCR on total RNA from various adult and fetal human tissues showed that *TMEM126A* is strongly expressed in the brain (whole), cerebellum, fetal brain, skeletal muscle, testis, fetal retinal pigmentary epithelium (RPE) and fetal retina (Figure 9). In situ hybridization on adult mouse retina (8 months) detected significant levels of specific mRNA in the ganglion cell layer (GCL), the optic nerve head (ON), the outer plexiform layer (OPL) and in the outer ellipsoide (oe) length of photoreceptor inner segments (IS). Faint to no labelling was noted in the outer nuclear layer (ONL) and photoreceptor outer segments. Immunolocalization of the mitochondria-specific alpha-subunit of the ATP synthase (ATP5A) on retinal sections of the same animal showed the same pattern of expression, supporting the view that *TMEM126A* transcripts colocalized with mitochondria (Figure 3). This result suggests that similarly to the dynamin-related GTPase protein Mgm1, of which OPA1 is the human ortholog, *TMEM126A* is a mitochondria localized mRNA (MLR) proteins and may be essential in early nucleation process of large mitochondrial complexes.¹³⁻¹⁶
In contrast to *OPA1* mutations, we found no fragmentation of the mitochondrial network and/or in depletions of the mitochondrial DNA (mtDNA)^{17, 18} in the fibroblasts of patients III6-Family 1 and V1 - Family 2 (data not shown) suggesting that *TMEM126A* and *OPA1* may not be functionally related.

Recently, mutations in an other TMEM protein have been reported to cause neonatal mitochondrial encephalocardiomyopathy.¹² Interestingly, the absence of TMEM70 in patients resulted in the loss of ATP synthase¹² which subunits have also been shown to be MLR proteins.¹⁶
As a probable MLR protein, TMEM126A may be important to the assembly of a protein complex required for the function retinal ganglion cells in higher eukaryotes. In other tissues, it is possible that another protein of the TMEM family could adequately substitute for the defective function of TMEM126A. It is also possible that TMEM126A may be required to accelerate the assembly of a protein complex. In the absence of TMEM126A, the complex would constitute slowly and only high energy-demanding retinal ganglion cells would be affected.
We have identified *TMEM126A* as a gene encoding a mitochondrial protein of higher eukaryotes which mutations are to our knowledge the first known genetic cause of non-syndromic autosomal recessive optic atrophy. This finding gives further support to the view that mitochondrial dysfunctions are a hallmark of optic neuropathies, suggesting that common therapeutic approaches may be developed to improve the long-term prognosis of patients whatever the gene involved.^{19, 20}

### Web resources

The URLs for data presented herein are as follows:
Online Mendelian Inheritance in Man (OMIM), http://www.ncbi.nlm.nih.gov/Omim/
Ensembl, http://www.ensembl.org
UCSC Genome Browser, http://genome.ucsc.edu
NCBI BLAST, http://www.ncbi.nlm.nih.gov/BLAST
Pfam Sanger Institute, http://pfam.sanger.ac.uk
PredictProtein, http://www.predictprotein.org

### REFERENCES

Throughout this application, various references describe the state of the art to which this invention pertains. The disclosures of these references are hereby incorporated by reference into the present disclosure.
1. Lev, D., Yanoov-Sharav, M., Watemberg, N., Leshinsky-Silver, E., Lerman-Sagie, T. (2002). White matter abnormalities in Leber's hereditary optic neuropathy due to the 3460 mitochondrial DNA mutation. Eur. J. Paediatr. Neurol. 6, 121-123.
2. Vinkler, C., Lev, D., Kalish, H., Watemberg, N., Yanoov-Sharav, M., Leshinsky-Silver, E., Lerman-Sagie, T. (2003). Familial optic atrophy with white matter changes. Am. J. Med. Genet. A. 121A, 263-265.
3. Yu-Wai-Man, P., Griffiths, P.G., Hudson, G., Chinnery, P.F. (2009). Inherited Mitochondrial Optic Neuropathies. J. Med. Genet. 46, 145-158.
4. Amati-Bonneau, P., Valentino, M.L., Reynier, P., Gallardo, M.E., Bornstein, B., Boissière, A., Campos, Y., Rivera, H., de la Aleja, J.G., Carroccia, R. et al.. (2008). OPA1 mutations induce mitochondrial DNA instability and optic atrophy 'plus' phenotypes. Brain 131, 338-51.
5. Barbet, F., Gerber, S., Hakiki, S., Perrault, I., Hanein, S., Ducroq, D., Tanguy, G., Dufier, J.L., Munnich, A., Rozet, J.M. et al. (2003). A first locus for isolated autosomal recessive optic atrophy (ROA1) maps to chromosome 8q. Eur. J. Hum. Genet. 11, 966-971.
6. Carelli, V., La Morgia, C., lommarini, L., Carroccia, R., Mattiazzi, M., Sangiorgi, S., Farne, S., Maresca, A., Foscarini, B., Lanzi, L., et al. (2007). Mitochondrial optic neuropathies: how two genomes may kill the same cell type? Biosci. Rep. 27, 173-184.
7. Calvo, S., Jain, M., Xie, X., Sheth, S.A., Chang, B., Goldberger, O.A., Spinazzola, A., Zeviani, M., Carr, S.A., Mootha, V.K. (2006). Systematic identification of human mitochondrial disease genes through integrative genomics. Nat. Genet. 38, 576-582.
8. Hanein, S., Perrault, I., Gerber, S., Delphin, N., Benezra, D., Shalev, S., Carmi, R., Feingold, J., Dufier, J.L., Munnich, A., et al. (2008). Population history and infrequent mutations: how old is a rare mutation? GUCY2D as a worked example. Eur. J. Hum. Genet. 16, 115-123.
9. Rustin, P., Chretien, D., Bourgeron, T., Gérard, B., Rötig, A., Saudubray, J.M., Munnich, A. (1994). Biochemical and molecular investigations in respiratory chain deficiencies. Clin. Chim. Acta. 228, 35-51.
10. Zanna, C., Ghelli, A., Porcelli, A.M., Karbowski, M., Youle, R.J., Schimpf, S., Wissinger, B., Pinti, M., Cossarizza, A., Vidoni, S., et al. (2008). OPA1 mutations associated with dominant optic atrophy impair oxidative phosphorylation and mitochondrial fusion. Brain 131, 352-367.
11. Smith, U.M., Consugar, M., Tee, L.J., McKee, B.M., Maina, E.N., Whelan, S., Morgan, N.V., Goranson, E., Gissen, P., Lilliquist, S., et al. (2006). The transmembrane protein meckelin (MKS3) is mutated in Meckel-Gruber syndrome and the wpk rat. Nat. Genet. 38, 191-196.
12. Cizkova, A., Stránecký, V., Mayr, J.A., Tesarová, M., Havlícková, V., Paul, J., Ivánek, R., Kuss, A.W., Hansíková, H., Kaplanová, V., et al. (2008). TMEM70 mutations cause isolated ATP synthase deficiency and neonatal mitochondrial encephalocardiomyopathy. Nat. Genet. 40,1288-1290.
13. Heinrich, P.C., Schmelzer, E., Northemann, W., Kaiser, C., Witt, I. (1982). Rat liver cytochrome c oxidase subunits IV and V: cell-free synthesis as larger molecular weight precursors, mRNA sizes and sites of synthesis. Prog. Clin. Biol. Res. 102, 149-159.
14. Rings, E.H., Büller, H.A., de Boer, P.A., Grand, R.J., Montgomery, R.K., Lamers, W.H., Charles, R., Moorman, A.F. (1992). Messenger RNA sorting in enterocytes. Co-localization with encoded proteins. FEBS. Lett. 300, 183-187.
15. Sylvestre, J., Vialette, S., Corral Debrinski, M., Jacq, C. (2003). Long mRNAs coding for yeast mitochondrial proteins of prokaryotic origin preferentially localize to the vicinity of mitochondria. Genome Biol. 4, R44.
16. Garcia, M., Darzacq, X., Delaveau, T., Jourdren, L., Singer, R.H., Jacq, C. (2007). Mitochondria-associated yeast mRNAs and the biogenesis of molecular complexes. Mol. Biol. Cell. 18, 362-368.
17. Frezza, C., Cipolat, S., Martins de Brito, O., Micaroni, M., Beznoussenko, G.V., Rudka, T., Bartoli, D., Polishuck, R.S., Danial, N.N., et al. (2006). OPA1 controls apoptotic cristae remodeling independently from mitochondrial fusion. Cell 126, 177-189.
18. Olichon, A., Landes, T., Arnauné-Pelloquin, L., Emorine, L.J., Mils, V., Guichet, A., Delettre, C., Hamel, C., Amati-Bonneau, P., Bonneau, D., et al. (2007). Effects of OPA1 mutations on mitochondrial morphology and apoptosis: relevance to ADOA pathogenesis. J. Cell. Physiol. 211, 423-4230.
19. Delletre, C., Lenaers, G., Griffoin, J.M., Gigarel, N., Lorenzo, C., Belenguer, P., Pelloquin, L., Grosgeorge, J., Turc-Carel, C., Perret, E., et al. (2000). Nuclear gene OPA1, encoding a mitochondrial dynamin-related protein, is mutated in dominant optic atrophy. Nat. Genet. 26, 207-210.
20. Chevrollier, A., Guillet, V., Loiseau, D., Gueguen, N., de Crescenzo, M.A., Verny, C., Ferre, M., Dollfus, H., Odent, S., Milea, D., et al. (2008). Hereditary optic neuropathies share a common mitochondrial coupling defect. Ann. Neurol. 63,794-798.
21. Bolte, S., Cordelières, F.P. (2006). A guided tour into subcellular colocalization analysis in light microscopy. J.Microsc. 224, 213-232.
22. Abecasis, G.R., Cherny, S.S., Cookson, W.O., Cardon, LR. (2002). Merlin-rapid analysis of dense genetic maps using sparse gene flow trees. Nat. Genet. 30, 97-101.

## Claims

1. A method for diagnosing or predicting a non syndromic autosomal recessive optic atrophy, or a risk of a non syndromic autosomal recessive optic atrophy, in a subject,
said method comprising detecting a mutation in the *TMEM126A* gene in a sample obtained from said subject,
wherein the presence of a homozygous *TMEM126A* mutation is indicative of a non syndromic autosomal recessive optic atrophy or of a risk of a non syndromic autosomal recessive optic atrophy,
wherein the *TMEM126A* mutation is a substitution of C by T at position 163 of SEQ ID NO: 1.

2. The method for diagnosing or predicting a non syndromic autosomal recessive optic atrophy, or a risk of a non syndromic autosomal recessive optic atrophy, according to claim 1 wherein said method is a prenatal method.

3. A transgenic non-human animal which is TMEM126A-deficient due to a mutation in the *TMEM126A* gene,
wherein said *TMEM126A* mutation is a substitution of C by T at position 163 of SEQ ID NO: 1.

## Patentansprüche

1. Verfahren zur Diagnose oder Vorhersage einer nicht-syndromischen autosomalen rezessiven optischen Atrophie oder eines Risikos für eine nicht-syndromische autosomale rezessive optische Atrophie in einem Subjekt, wobei das Verfahren die Detektion einer Mutation in dem *TMEM126A-*Gen in einer Probe, die diesem Patienten entnommen wurde, umfasst,
wobei die Präsenz einer homozygoten *TMEM126A-Mutation* eine nicht-syndromische autosomale rezessive optische Atrophie oder ein Risiko für eine nicht-syndromische autosomale rezessive optische Atrophie indiziert,
wobei die TMEM126A-Mutation eine Substitution von C durch T an der Position 163 von SEQ ID Nr. 1 ist.

2. Verfahren zur Diagnose oder Vorhersage einer nicht-syndromischen autosomalen rezessiven optischen Atrophie oder eines Risikos für eine nicht-syndromische autosomale rezessive optische Atrophie nach Anspruch 1, wobei das Verfahren ein pränatales Verfahren ist.

3. Transgenes nicht-humanes Tier, welches *TMEM126A*-defizient aufgrund einer Mutation in dem *TMEM126A*-Gen ist,
wobei die *TMEM126A*-Mutation eine Substitution von C durch T an Position 163 von SEQ ID Nr. 1 ist.

## Revendications

1. Procédé pour diagnostiquer ou prédire une atrophie optique récessive autosomique non syndromique, ou un risque d'une atrophie optique récessive autosomique non syndromique, chez un sujet, ledit procédé comprenant la détection d'une mutation dans le gène *TMEM126A* dans un échantillon obtenu dudit sujet,
dans lequel la présence d'une mutation homozygote de *TMEM126A* est indicatrice d'une atrophie optique récessive autosomique non syndromique ou d'un risque d'une atrophie optique récessive autosomique non syndromique,
dans lequel la mutation de *TMEM126A* est une substitution de C par T à la position 163 de SEQ ID NO :1.

2. Procédé pour diagnostiquer ou prédire une atrophie optique récessive autosomique non syndromique, ou un risque d'une atrophie optique récessive autosomique non syndromique, selon la revendication 1 dans lequel ledit procédé est un procédé prénatal.

3. Animal non humain transgénique qui est déficient en TMEM126A du fait d'une mutation dans le gène *TMEM126A,*
dans lequel ladite mutation de *TMEM126A* est une substitution de C par T à la position 163 de SEQ ID NO :1.


### SEQUENCE LISTING

<110> INSERM
<120> Method for diagnosing or predicting a non syndromic autosomal recessive optic atrophy, or a risk of a non syndromic autosomal recessive optic atrophy
<130> BEP090188
<160> 29
<170> PatentIn version 3.3
<210> 1
   <211> 588
   <212> DNA
   <213> Homo sapiens
<400> 1
   atggaaaatc ataaatccaa taataaggaa aacataacaa ttgttgatat atccagaaaa     60
   attaaccagc ttccagaagc agaaaggaat ctacttgaaa atggatcggt ttatgttgga    120
   ttaaatgctg ctctttgtgg cctcatagca aacagtcttt ttcgacgcat cttgaatgtg    180
   acaaaggctc gcatagctgc tggcttacca atggcaggga taccttttct tacaacagac    240
   ttaacttaca gatgttttgt aagttttcct ttgaatacag gtgatttgga ttgtgaaacc    300
   tgtaccataa cacggagtgg actgactggt cttgttattg gtggtctata ccctgttttc    360
   ttggctatac ctgtaaatgg tggtctagca gccaggtatc aatcagctct gttaccacac    420
   aaagggaaca tcttaagtta ctggattaga acttctaagc ctgtctttag aaagatgtta    480
   tttcctattt tgctccagac tatgttttca gcataccttg ggtctgaaca atataaacta    540
   cttataaagg cccttcagtt atctgaacct ggcaaagaaa ttcactga                588
<210> 2
   <211> 195
   <212> PRT
   <213> Homo sapiens
<400> 2
   Met Glu Asn His Lys Ser Asn Asn Lys Glu Asn Ile Thr Ile Val Asp
   1               5                   10                  15
   Ile Ser Arg Lys Ile Asn Gln Leu Pro Glu Ala Glu Arg Asn Leu Leu
               20                  25                  30
   Glu Asn Gly Ser Val Tyr Val Gly Leu Asn Ala Ala Leu Cys Gly Leu
           35                  40                  45
   Ile Ala Asn Ser Leu Phe Arg Arg Ile Leu Asn Val Thr Lys Ala Arg
       50                  55                  60
   Ile Ala Ala Gly Leu Pro Met Ala Gly Ile Pro Phe Leu Thr Thr Asp
   65                  70                  75                  80
   Leu Thr Tyr Arg Cys Phe Val Ser Phe Pro Leu Asn Thr Gly Asp Leu
                   85                  90                  95
   Asp Cys Glu Thr Cys Thr Ile Thr Arg Ser Gly Leu Thr Gly Leu Val
               100                 105                 110
   Ile Gly Gly Leu Tyr Pro Val Phe Leu Ala Ile Pro Val Asn Gly Gly
           115                 120                 125
   Leu Ala Ala Arg Tyr Gln Ser Ala Leu Leu Pro His Lys Gly Asn Ile
       130                 135                 140
   Leu Ser Tyr Trp Ile Arg Thr Ser Lys Pro Val Phe Arg Lys Met Leu
   145                 150                 155                 160
   Phe Pro Ile Leu Leu Gln Thr Met Phe Ser Ala Tyr Leu Gly Ser Glu
                   165                 170                 175
   Gln Tyr Lys Leu Leu Ile Lys Ala Leu Gln Leu Ser Glu Pro Gly Lys
               180                 185                 190
   Glu Ile His
           195
<210> 3
   <211> 21
   <212> DNA
   <213> Artificial
<220>
   <223> primer
<400> 3
   acagagtcac gtgtgagcca a 21
<210> 4
   <211> 20
   <212> DNA
   <213> Artificial
<220>
   <223> primer
<400> 4
   ccattgacgg gtatagccaa 20
<210> 5
   <211> 20
   <212> DNA
   <213> Artificial
<220>
   <223> primer
<400> 5
   ccattgacgg gtatagccaa 20
<210> 6
   <211> 21
   <212> DNA
   <213> Artificial
<220>
   <223> primer
<400> 6
   acagagtcac gtgtgagcca a 21
<210> 7
   <211> 23
   <212> DNA
   <213> Artificial
<220>
   <223> primer
<400> 7
   taatacgact cactataggg aga 23
<210> 8
   <211> 20
   <212> DNA
   <213> Artificial
<220>
   <223> primer
<400> 8
   taaccagctt ccagaagcag 20
<210> 9
   <211> 22
   <212> DNA
   <213> Artificial
<220>
   <223> primer
<400> 9
   gtgaatttct ttgccaggtt ca 22
<210> 10
   <211> 20
   <212> DNA
   <213> Artificial
<220>
   <223> primer
<400> 10
   accagctaac caacgacaaa 20
<210> 11
   <211> 20
   <212> DNA
   <213> Artificial
<220>
   <223> primer
<400> 11
   tgctgttcct gaatctgagc 20
<210> 12
   <211> 17
   <212> DNA
   <213> Artificial
<220>
   <223> primer
<400> 12
   tgccgggagc ctaaaat 17
<210> 13
   <211> 20
   <212> DNA
   <213> Artificial
<220>
   <223> primer
<400> 13
   actcaccctg cagatcttag 20
<210> 14
   <211> 20
   <212> DNA
   <213> Artificial
<220>
   <223> primer
<400> 14
   gatacatgta tacattgtgt 20
<210> 15
   <211> 20
   <212> DNA
   <213> Artificial
<220>
   <223> primer
<400> 15
   gttaatcatt ttgacatgtt 20
<210> 16
   <211> 20
   <212> DNA
   <213> Artificial
<220>
   <223> primer
<400> 16
   tcactcaact cctgaacctc 20
<210> 17
   <211> 18
   <212> DNA
   <213> Artificial
<220>
   <223> primer
<400> 17
   aagatctacc ccagtgcg 18
<210> 18
   <211> 20
   <212> DNA
   <213> Artificial
<220>
   <223> primer
<400> 18
   gcttcctcat tccctctctg 20
<210> 19
   <211> 18
   <212> DNA
   <213> Artificial
<220>
   <223> primer
<400> 19
   tagcctattg tgggaccc 18
<210> 20
   <211> 20
   <212> DNA
   <213> Artificial
<220>
   <223> primer
<400> 20
   cttctcagcc caaagccgct 20
<210> 21
   <211> 20
   <212> DNA
   <213> Artificial
<220>
   <223> primer
<400> 21
   cgtcgtgctt ctcctgacac 20
<210> 22
   <211> 22
   <212> DNA
   <213> Artificial
<220>
   <223> primer
<400> 22
   cagattagct ctacagtatt at 22
<210> 23
   <211> 20
   <212> DNA
   <213> Artificial
<220>
   <223> primer
<400> 23
   ccttctcaca ttccatgttg 20
<210> 24
   <211> 20
   <212> DNA
   <213> Artificial
<220>
   <223> primer
<400> 24
   gggatagatg tcgtatccag 20
<210> 25
   <211> 21
   <212> DNA
   <213> Artificial
<220>
   <223> primer
<400> 25
   attacagcat acagtacttg g 21
<210> 26
   <211> 24
   <212> DNA
   <213> Artificial
<220>
   <223> primer
<400> 26
   caattacatt tgatatatta cctg 24
<210> 27
   <211> 20
   <212> DNA
   <213> Artificial
<220>
   <223> primer
<400> 27
   gggtaacatt gcctttggtc 20
<210> 28
   <211> 22
   <212> DNA
   <213> Artificial
<220>
   <223> primer
<400> 28
   tttatcttaa gacttctagg ac 22
<210> 29
   <211> 20
   <212> DNA
   <213> Artificial
<220>
   <223> primer
<400> 29
   ccttttgttg taggtcccag 20